(19) Europäisches Patentamt
European Patent Office
Office européen des brevets

(11) **EP 2 708 892 B1**

(12) **EUROPÄISCHE PATENTSCHRIFT**

(45) Veröffentlichungstag und Bekanntmachung des Hinweises auf die Patenterteilung:
**15.04.2015 Patentblatt 2015/16**

(51) Int Cl.:
***G01N 33/86*** (2006.01)  ***G01N 33/50*** (2006.01)

(21) Anmeldenummer: **12184775.0**

(22) Anmeldetag: **18.09.2012**

(54) **Verfahren zur Bestimmung Plättchen-assoziierter Analyten**

Method for the determination of platelet associated analytes

Méthode pour la détermination d'analytes associés aux thrombocytes

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**19.03.2014 Patentblatt 2014/12**

(73) Patentinhaber: **Siemens Healthcare Diagnostics Products GmbH**
**35041 Marburg (DE)**

(72) Erfinder: **Patzke, Juergen, Dr.**
**35043 Marburg (DE)**

(56) Entgegenhaltungen:
**WO-A1-2012/098237**

- **RODEGHIERO F ET AL: "Platelet von Willebrand factor assay: results using two methods for platelet lysis", THROMBOSIS RESEARCH, TARRYTOWN, NY, US, Bd. 59, Nr. 2, 15. Juli 1990 (1990-07-15), Seiten 259-267, XP026359922, ISSN: 0049-3848 [gefunden am 1990-07-15]**
- **CHRISTOPHE DE ROMEUF ET AL: "INTEREST OF A SIMPLE AND FAST METHOD FOR PLATELET VON WILLEBRAND FACTOR CHARACTERIZATION", THROMBOSIS RESEARCH, Bd. 83, Nr. 4, 1. August 1996 (1996-08-01), Seiten 287-298, XP55041098, ISSN: 0049-3848, DOI: 10.1016/0049-3848(96)00137-5**

- **THUY L P ET AL: "Identification of platelet receptors for bovine von Willebrand factor on human platelets by a new platelet receptor test", BIOCHIMICA ET BIOPHYSICA ACTA - GENERAL SUBJECTS, ELSEVIER SCIENCE PUBLISHERS, NL, Bd. 678, Nr. 2, 4. Dezember 1981 (1981-12-04), Seiten 187-193, XP025852901, ISSN: 0304-4165, DOI: 10.1016/0304-4165(81)90205-1 [gefunden am 1981-12-04]**
- **A. R. RECHNER: "Platelet function testing in clinical diagnostics", HÄMOSTASEOLOGIE, Bd. 31, Nr. 2, 9. Dezember 2010 (2010-12-09), Seiten 79-87, XP55012141, ISSN: 0720-9355, DOI: 10.5482/ha-1133**
- **NYMAN ET AL: "Von Willebrand factor dependent platelet aggregation and adsorption of factor viii related antigen by collagen", THROMBOSIS RESEARCH, TARRYTOWN, NY, US, Bd. 17, Nr. 1-2, 1. Januar 1980 (1980-01-01), Seiten 209-214, XP026360156, ISSN: 0049-3848 [gefunden am 1980-01-01]**
- **TAKAHASHI ET AL: "Studies on the pathophysiology and treatment of von willebrand's disease. IV. Mechanism of increased ristocetin-induced platelet aggregation in von willebrand's disease", THROMBOSIS RESEARCH, TARRYTOWN, NY, US, Bd. 19, Nr. 6, 15. September 1980 (1980-09-15), Seiten 857-867, XP022876061, ISSN: 0049-3848, DOI: 10.1016/0049-3848(80)90013-4 [gefunden am 1980-09-15]**

**Beschreibung**

Die vorliegende Erfindung liegt auf dem Gebiet der Gerinnungsdiagnostik und betrifft ein in vitro-Verfahren zur Bestimmung Plättchen-assoziierter Analyten in einer Probe.

Physiologische Vorgänge, die einerseits die Fluidität des Blutes im Gefäßsystem gewährleisten und andererseits durch die Bildung von Blutgerinnseln die Vermeidung von extravasalen Blutverlusten sicherstellen, werden unter dem Begriff Hämostase zusammengefasst. An der Regulation der Hämostase sind eine Vielzahl von Proteinfaktoren beteiligt sowie auch zelluläre Komponenten, wie z.B. die Plättchen (synonym: Blutplättchen, Thrombozyten). Im Falle einer Gefäßverletzung kommt es zuerst zur Anlagerung von Plättchen an das subendotheliale Kollagen. Diese Adhäsion wird durch Adhäsivproteine, wie den von-Willebrand-Faktor (VWF) vermittelt. Während des Adhäsionsvorgangs werden die Plättchen aktiviert und schütten Mediatoren aus ihren Granulae aus, wodurch die Aggregation weiterer Plättchen sowie eine Verstärkung der Aktivierung induziert wird. Auf diese Weise erfolgt ein primärer Gefäßwandverschluss (primäre Hämostase), der erst durch weitere Reaktionen des plasmatischen Gerinnungssystems (sekundäre Hämostase) stabilisiert wird. Fehlregulationen dieser Prozesse können zu einer Thromboseneigung oder einer Blutungsneigung führen und je nach Schweregrad lebensbedrohliche Folgen haben.

Plättchen enthalten eine Vielzahl von intrazellulären Inhaltsstoffen, die physiologisch wichtige Funktionen haben. Wichtigster Speicherort für verschiedene Substanzen, wie z.B. Plättchenfaktor 4 (PF4), Fibrinogen, Faktor V, Faktor XIII, von Willebrand Faktor (VWF), Fibronektin, Calciumionen sowie Plättchen-Wachstumsfaktoren, wie z.B. PDGF (platelet derived growth factor), sind die alpha-Granulae und die dichten Granula im Zytoplasma der Plättchen. Werden die Plättchen z.B. infolge einer Gefäßverletzung aktiviert, werden die Inhaltsstoffe aus den Granulae sezerniert, wodurch wiederum für den Fortgang der Gerinnungsreaktion wichtige Prozesse angestossen werden. Andere Substanzen, wie z.B. Faktor XIII finden sich im Zytosol der Plättchen. Wiederum andere Substanzen sind in oder auf der Zellmembran der Plättchen gebunden, inbesondere Rezeptorproteine wie ADP-Rezeptor, Thromboxan-Rezeptor, Thrombin-Rezeptor oder die Glykoproteine Ia, Ib, IIb und IIIa. Weiterhin binden viele Proteine, wie z.B. Faktor X, Faktor IX und Faktor VIII, infolge der Plättchenaktivierung an die Zellmembran der Plättchen. VWF bindet infolge von Schärstress oder auf Grund von molekularen Defekten spontan an den GPIb-Rezeptor der Plättchen. Für die VWF-spaltende Protease ADAMTS-13 ist ein Plättchen-assoziierter Anteil ebenfalls bekannt.

Viele dieser Plättchen-assoziierten Substanzen und ihre regulierte Freisetzung spielen eine wichtige Rolle in der Hämostase. Dies wird auch daran deutlich, dass eine Reihe von Krankheitsbildern bekannt ist, die auf eine Störung der Granulae-Funktion in den Plättchen zurückzuführen ist. Eine bekannte Erkrankung ist das sog. Gray Platelet Syndrom (GPS), eine erbliche Blutungsstörung verursacht durch verminderte oder fehlende alpha-Granulae. Eine andere bekannte Erkrankung ist die sog. Quebec Platelet Disorder (QPD), ebenfalls eine erbliche Blutungsstörung, die durch eine übermäßige Degradation von alpha-granulären Inhaltsstoffen verursacht wird. Die Erkennung von Defizienzen oder Dysfunktionalitäten von Plättchen-assoziierten Substanzen bzw. die Erkennung von Defekten bei der Ausschüttung dieser Substanzen aus den Plättchen-Granulae ist daher von hoher diagnostischer Relevanz.

Eine weitere bekannte Erkrankung ist das sog. von Willebrand Syndrom (von Willebrand disease, VWD), eine erbliche oder erworbene Blutungsstörung. Da ein von Willebrand Syndrom durch unterschiedliche Störungen des VWF hervorgerufen sein kann, werden verschiedene Typen unterschieden. Beim Typ 3 VWD fehlt das VWF-Antigen (VWF:Ag) sowohl im Plasma als auch in den alpha-Granulae der Plättchen vollständig. Beim Typ 1 VWD, für das eine verminderte plasmatische VWF-Antigen-Konzentration kennzeichnend ist, werden zwei Subtypen differenziert, die sich dadurch unterscheiden, dass in dem einen Subtyp die alpha-granuläre VWF-Antigen-Konzentration in den Plättchen ebenfalls reduziert ist, während in dem anderen Subtyp die alpha-granuläre VWF-Antigen-Konzentration in den Plättchen normal ist.

Da bei den verschiedenen Typen des von Willebrand Syndroms unterschiedliche Therapien eingesetzt werden, erfordert jeder Einzelfall eine möglichst genaue Klassifikation. Daher ist es wünschenswert, nicht nur die VWF-Antigen-Konzentration oder - Aktivität im Plasma eines Patienten zu bestimmen, sondern auch die VWF-Antigen-Konzentration oder -Aktivität in den Plättchen des Patienten.

Bekannte Verfahren zur Bestimmung von Plättchen-assoziierten Substanzen sind technisch aufwändig und nicht für eine routinemäßige Abarbeitung im klinischen Labor geeignet. Grundsätzlich problematisch ist, dass viele diagnostisch relevante Plättchen-assoziierte Substanzen, wie z.B. VWF, nicht exklusiv in den Plättchen, sondern auch im Plasma vorkommen. Üblicherweise werden zur Analyse von intrazellulären Plättchen-Analyten Plättchenlysate aus frischen gewaschenen Plättchen hergestellt. Dazu wird plättchenreiches Plasma zur Entfernung der Plasmabestandteile mehrmals in einer Waschlösung suspendiert, zentrifugiert und anschließend werden die Plättchen durch Zugabe eines Detergenz-haltigen Puffers lysiert. In dem so erhaltenen plasmafreien Plättchenlysat wird dann die Menge oder die Aktivität eines gewünschten Analyten bestimmt.

De Romeuf & Mazurier beschreiben ein vereinfachtes Verfahren zur Bestimmung von Plättchen-VWF, bei dem zumindest auf die Waschschritte verzichtet wird. Das vereinfachte Verfahren besteht darin, dass plättchenreiches Plasma nur einmal zentrifugiert wird, der Überstand sorgfältig abgenommen wird, und das Zellpellet in einem Detergenz-haltigen Puffer resuspendiert wird. In dem so erhaltenen Plättchenlysat wird dann die Menge oder die Aktivität des Plättchen-VWF bestimmt (De Romeuf, C. & Mazurier, C. Interest of a simple and fast method for platelet von Willebrand factor characterization. Thrombosis Research 1996, 83(4): 287-298).

Auch dieses vereinfachte Verfahren ist für eine routinemäßige Abarbeitung im klinischen Labor und insbesondere nicht für eine automatische Testdurchführung geeignet, da die Probenvorbereitung immer noch erfordert, dass das Probenmaterial (plättchenreiches Plasma) zentrifugiert und der Überstand sorgfältig abgenommen werden muss. Zumindest auf dem Gebiet der automatischen Gerinnungsanalyzer sind keine Systeme bekannt, die eine Probe zentrifugieren und präzise den Überstand von einem Zellpellet abnehmen könnten.

Rodeghiero, F. et al. beschreiben ein Verfahren zur Bestimmung von Plättchen-VWF in Plättchenlysaten, die durch Lyse der Plättchen durch Zugabe von Detergenz oder Glycerol erhalten wurden (Rodeghiero, F. et al., Platelet von Willebrand factor assay: results using two methods for platelet lysis. Thrombosis Research 1990, 59: 259-267).

Thuy, L.P. et al. beschreiben ein Verfahren zur Bestimmung der Aktivität des Plättchenezeptors für bovinen VWF in humanen Plättchenextrakten, die ebenfalls durch Zugabe von Detergenz erhalten wurden (Thuy, L.P. et al., Identification of platelet receptors for bovine von Willebrand factor on human platelets by a new platelet receptor test. Biochimica et Biophysica Acta 1981, 678: 187-193).

Der vorliegenden Erfindung lag daher die Aufgabe zugrunde, ein Verfahren zur Bestimmung der Menge oder der Aktivität eines Plättchen-assoziierten Analyten in einer Probe bereit zu stellen, welches auf eine aufwändige Probenvorbereitung, insbesondere auf zusätzliche Zentrifugations- und Waschschritte verzichtet und welches für eine routinemäßige Abarbeitung im klinischen Labor und insbesondere zur Abarbeitung auf einem automatisierten Testsystem geeignet ist.

Die Aufgabe wird dadurch gelöst, dass ein Verfahren mit den folgenden Schritten durchgeführt wird:

a) Bereitstellung eines ersten Testansatzes enthaltend plättchenreiches Plasma eines Individuums und ein Detergenz,

b) Bereitstellung eines zweiten Testansatzes enthaltend entweder

i) plättchenreiches Plasma des Individuums und kein Detergenz oder
ii) plättchenarmes Plasma des Individuums und kein Detergenz oder
iii) plättchenarmes Plasma des Individuums und ein Detergenz,

c) Messung der Menge oder der Aktivität des Analyten im ersten und im zweiten Testansatz und
d) Vergleich der Messergebnisse,

wobei die Differenz zwischen den Messergebnissen der Menge oder der Aktivität des Plättchen-assoziierten Analyten entspricht.

Der Begriff "Plättchen-assoziierter Analyt" ist breit zu verstehen und umfasst sowohl intrazelluläre Inhaltsstoffe von Plättchen, wie z.B. Inhaltsstoffe der Plättchen-Granulae oder des Cytosols, extrazelluläre Substanzen, die der äußeren Plättchenoberfläche anhaften, wie z.B. VWF oder Fibrinogen, als auch Membranbestandteile, die in der Zellmembran der Plättchen verankert sind, wie z.B. Transmembranrezeptoren. Der Begriff "Plättchen-assoziierter Analyt" umfasst alle Arten von Substanzen, wie z.B. Peptide, Proteine (z.B. Faktor XIII, VWF, GPIb-Rezeptor, Fibrinogen, D-Dimer), Lipide, Ionen (z.B. Calciumionen), Nukleotide (z.B. ADP und ATP), Nukleinsäuren (z.B. DNA, RNA und miRNA) und langkettige Polyphosphate (n> 50), die in isolierten, bevorzugt humanen Plättchen enthalten sind.

Der Begriff "plättchenreiches Plasma" (PRP) umfasst eine Plasmaprobe eines Individuums, bevorzugt eine Plasmaprobe eines menschlichen Individuums, die mindestens 10.000 Plättchen pro Mikroliter [$\mu$L] Probe enthält. Dem Fachmann sind diverse Verfahren zur Gewinnung von plättchenreichem Plasma aus einer Vollblutprobe bekannt. Ein übliches Verfahren funktioniert wie folgt: Eine antikoagulierte Vollblutprobe wird bei 170 g für 15 Minuten oder bei 180 g für 10 Minuten zentrifugiert. Dadurch wird erreicht, dass sich eine untere Schicht aus roten und weißen Blutzellen bildet und darüber eine Schicht plättchenreiches Plasma. Letzteres wird vorsichtig ohne Vermischung mit der unteren Schicht entnommen.

Plättchenarmes Plasma (PPP) hingegen, das üblicherweise gemeint ist, wenn der Begriff "Plasma" verwendet wird, ist idealerweise plättchenfrei, muss aber in jedem Fall weniger als 10.000 Plättchen pro Mikroliter [$\mu$L] Probe enthalten. Dem Fachmann sind diverse Verfahren zur Gewinnung von plättchenarmem Plasma aus einer Vollblutprobe bekannt. Ein übliches Verfahren funktioniert wie folgt: Eine antikoagulierte Vollblutprobe wird bei 1500 g für mindestens 15 Minuten zentrifugiert. Dadurch wird erreicht, dass sich eine untere Schicht aus roten und weißen Blutzellen und aus Plättchen bildet und darüber eine Schicht plättchenarmes Plasma. Letzteres wird vorsichtig ohne Vermischung mit der unteren Schicht entnommen.

Der Begriff "Detergenz" umfasst oberflächenaktive Substanzen, insbesondere nichtionischen Tenside, die in der Lage sind, die Löslichkeit von zellulären Lipidmembranen in einem wässrigen Medium zu erhöhen. Detergenzien permeabilisieren bekanntermaßen zelluläre Membranen, so dass intrazelluläre Bestandteile oder Transmembranbestandteile in das umgebende Medium abgegeben werden bzw. für Bindungspartner, die zum Nachweis der zellulären Bestandteile verwendet werden, leichter zugänglich sind. Geeignete Detergenzien zur Permeabilisierung von zellulären Lipidmembranen bzw. zur Lyse von Zellen wie z.B. auch von Plättchen sind z.B. Polyethylenglycol-[4- (1,1,3,3-tetramethylbutyl) phenyl]-ether (Handelsname TRITON® X-100), Polyoxyethylen(20)-sorbitan-monolaurat (Handelsname TWEEN® 20), Polidocanol (Handelsname THESIT®, Synonyme: Polyethylene glycol dodecyl ether, Polyethylene glycol 400 dodecyl ether), Glycerin, Saponin, Digitonin, Filipin, Octylglucosid, Dodecylsulfate, Zonyl® und Deoxycholat.

Das erfindungsgemäße Verfahren zur Bestimmung der Menge oder der Aktivität eines Plättchen-assoziierten Analyten in einer Probe eines Individuums umfasst die Bereitstellung eines ersten Testansatzes enthaltend plättchenreiches Plasma des Individuums und ein Detergenz. Bevorzugterweise wird dem plättchenreichen Plasma das Detergenz in einer solchen Menge zugesetzt, dass das Detergenz im Testansatz in einem Volumenanteil von 0,1 bis 1,6 %, besonders bevorzugt von 0,2 bis 0,8 % enthalten ist. Durch die Zugabe von Analyt-spezifischen Nachweisreagenzien, wie z.B. gelabelten Bindungspartnern mit Spezifität für den nachzuweisenden Analyten, kann die Menge oder die Aktivität des Analyten in dem Testansatz bestimmt werden. Die im ersten Testansatz bestimmte Menge oder Aktivität des Analyten entspricht der Summe aus der Menge oder Aktivität des Analyten, die im Plasma vorhanden ist (Plasmaanteil), und der Menge oder Aktivität desselben Analyten, der in oder an den Plättchen vorhanden ist (Plättchenanteil).

Das erfindungsgemäße Verfahren umfasst ferner die Bereitstellung eines zweiten Testansatzes enthaltend entweder

i) plättchenreiches Plasma des Individuums und kein Detergenz oder
ii) plättchenarmes Plasma des Individuums und kein Detergenz oder
iii) plättchenarmes Plasma des Individuums und ein Detergenz.

Bevorzugterweise wird in Alternative iii) dem plättchenarmen Plasma das Detergenz in einer solchen Menge zugesetzt, dass das Detergenz in einer Endkonzentration mit einem Volumenanteil von 0,1 bis 1,6 %, besonders bevorzugt in einem Volumenanteil von 0,2 bis 0,8 % im Testansatz enthalten ist. Besonders bevorzugt wird in Alternative iii) dem plättchenarmen Plasma das Detergenz in der Menge zugesetzt, so dass das Detergenz in derselben Endkonzentration enthalten ist wie in dem ersten Testansatz. Durch die Zugabe von denselben Analyt-spezifischen Nachweisreagenzien wie im ersten Testansatz, wie z.B. gelabelten Bindungspartnern mit Spezifität für den nachzuweisenden Analyten, kann die Menge oder die Aktivität des Analyten in dem zweiten Testansatz bestimmt werden. Die im zweiten Testansatz bestimmte Menge oder Aktivität des Analyten entspricht der Menge oder Aktivität des Analyten, die im Plasma vorhanden ist (plasmatischer Analyt). Die Menge oder Aktivität desselben Analyten, der in den Plättchen vorhanden ist (Plättchen-assoziierter Analyt), wird nicht bestimmt, weil entweder kein Detergenz verwendet wird, das den Plättchenanteil des Analyten zugänglich machen würde (Alternative i) oder weil nur plättchenarmes Plasma verwendet wird, das keine ausreichende Menge an Plättchen enthält (Alternativen ii und iii).

Das erfindungemäße Verfahren umfasst ferner den Vergleich der Messergebnisse, also den Vergleich des Messergebnisses für den ersten Testansatz (Menge oder Aktivität des plasmatischen und des Plättchen-assoziierten Analyten) mit dem Messergebnis für den zweiten Testansatz (Menge oder Aktivität des plasmatischen Analyten). Die Differenz zwischen den Messergebnissen entspricht der Menge oder der Aktivität des Plättchen-assoziierten Analyten. So kann beispielsweise durch Subtraktion des Messergebnisses für den zweiten Testansatz vom Messergebnis für den ersten Testansatz die Menge oder Aktivität des Plättchen-assoziierten Analyten bestimmt werden.

Die Messung der Menge oder Aktivität des Analyten im ersten und zweiten Testansatz kann mit Hilfe jeder Nachweismethode erfolgen, die für den Nachweis des Analyten geeignet ist

Die Messung der Menge oder Aktivität des Analyten kann z.B. in einem heterogenen Bindungstest erfolgen. Heterogene Bindungsteste sind durch einen oder mehrere Trennungsschritte und/oder Waschschritte gekennzeichnet. Die Trennung kann beispielsweise durch Immunfällung, Fällung mit Substanzen wie Polyethylenglykol oder Ammoniumsulfat, Filtration, magnetische Abtrennung, Anbindung an eine Festphase erfolgen. Ein solche Festphase besteht aus porösem und/oder nicht porösem, in der Regel wasserunlöslichem Material. Sie kann die unterschiedlichsten Formen aufweisen wie z.B.: Gefäß, Röhrchen, Mikrotitrationsplatte, Kugel, Mikropartikel, Stäbchen, Streifen, Filter- oder Chromatographiepapier etc. Bei heterogenen Bindungstesten im Sandwichformat ist in der Regel ein erster Analyt-spezifischer Bindungspartner, z.B. ein monoklonaler oder polyklonaler Antikörper oder ein Analyt-bindendes Antikörperfragment oder ein Oligonukleotid an eine Festphase gebunden und dient zur Abtrennung des Bindungskomplexes Analyt/Analyt-spezifischer Bindungspartner von der flüssigen Phase, während ein zweiter Analyt-spezifischer Bindungspartner zum Nachweis des Bindungskomplexes ein nachweisbares Label (z.B. ein Enzym, ein Fluoreszenz- oder Chemilumineszenzlabel etc.) trägt. Man unterteilt diese Testverfahren weiter in sogenannte Einschritt-Sandwich-Teste, bei denen die beiden spezifischen Bindungspartner simultan mit der Probe inkubiert werden und in Zweischritt-Sandwich-Teste, bei denen die Probe zunächst mit dem Festphasenreagenz inkubiert wird und nach einem Trenn- und Waschschritt der festphasengebundene Bindungskomplex aus Analyt und Analyt-spezifischem Bindungspartner mit dem Nachweisreagenz inkubiert wird. Ein klassisches Beispiel für einen heterogenen Bindungstest ist der ELISA-Test.

Die Messung der Menge oder Aktivität des Analyten kann auch in einem homogenen Bindungstest erfolgen. Bei homogenen Bindungstesten erfolgt keine Trennung von freien und gebundenen Analyt-spezifischen Bindungspartnern. Der Testansatz, welcher die Analyt-spezifischen Bindungspartner, die signalbildenden Komponenten und die Probe enthält, wird nach oder sogar während der Bindungsreaktion ohne einen weiteren Trenn- oder Waschschritt gemessen und das entsprechende Messsignal bestimmt. Beispiele für homogene Immunoassays sind viele turbidimetrische oder nephelometrische Methoden, wobei die zum Nachweis verwendeten Analyt-spezifischen Bindungspartner mit Latexpartikeln assoziiert sein können. Beispiele für homogenen Testformate sind EMIT®-Teste, CEDIA®-Teste; Fluoreszenz-Polarisations-Immunoassays, Luminescent Oxygen Channeling Immunoassays ("LOCI", siehe EP-A2-515194), T2MR (T2 Biosystems, Inc., siehe US-A1-20120164644) etc. Bei einem homogenen Sandwich-Immunoassay, wie z.B. einem nephelometrischen Latextest, werden die Bindungspartner, z.B. monoklonale oder polyklonale Antikörper oder ein Analyt-bindendes Antikörperfragment oder ein Oligonukleotid, mit der Probe zusammen inkubiert und die Messung des Signals während und/oder nach der Inkubation durchgeführt, ohne dass ein Trenn- oder Waschschritt vor der Messung durchgeführt wird.

In einer bevorzugten Ausführungsform des erfindungsgemäßen Verfahrens erfolgt die Messung der Menge oder Aktivität des Analyten im ersten und zweiten Testansatz mit Hilfe eines Latexagglutinationstests. Dazu wird jeder Testansatz mit einer partikulären Festphase vermischt, bevorzugt mit Latexpartikeln, die mit mindestens einem Analyt-spezifischen Bindungspartner assoziiert sind und die Agglutination der partikulären Festphase wird optisch gemessen. Alternativ wird jeder Testansatz mit magnetischen Partikeln vermischt, welche Bindungspartner auf der Oberfläche haben, die bei Präsenz des Analyten die Aggregation der Partikel verändern. Der Reaktionsansatz wird einem magnetischem Feld ausgesetzt, und die Agglutination der partikulären Festphase wird anhand der T2-Relaxationsrate des Reaktionsansatzes bestimmt.

Die Messung der Menge oder Aktivität des Analyten kann auch in einem kompetitiven Bindungstest erfolgen. Bei einem kompetitiven Bindungstest konkurrieren Proben-Analyt und Reagenz-Analyt, beispielsweise ein gelabelter Analyt, um die Bindung an eine limitierte Anzahl von Analyt-spezifischen Bindungspartnern. Beispiele zur Illustration des Prinzips: (i) Proben-Analyt konkurriert mit Reagenz-Analyt, der mit einer Komponente eines signalbildenden System assoziiert ist, um die Bindung an Festphasen-assoziierte Analyt-spezifische Bindungspartner oder (ii) Proben-Analyt konkurriert mit Festphasen-assoziiertem Reagenz-Analyt um die Bindung an Analyt-spezifische Bindungspartner, die mit einer Komponente eines signalbildenden Systems assoziiert sind.

In einer bevorzugten Ausführungsform des erfindungsgemäßen Verfahrens wird die Menge oder die Aktivität von Plättchen-assoziiertem VWF gemessen. Die Messung der Menge von VWF-Antigen (VWF:Ag) in einem Testansatz kann mit Hilfe eines heterogenen oder homogenen Bindungstests erfolgen, bei dem mindestens ein VWF-spezifischer Bindungspartner, bevorzugt ein monoklonaler oder polyklonaler Antikörper oder ein VWFbindendes Antikörperfragment verwendet wird. Die Messung der VWF-Aktivität (VWF Ac) in einem Testansatz kann mit Hilfe eines klassischen Ristocetin-Cofaktor-Assays (VWF:Rco) bestimmt werden. Hierzu wird der Testansatz mit Ristocetin und fixierten Plättchen vermischt und die Agglutination der Plättchen wird bestimmt. Alternativ kann die VWF-Aktivität auch anhand der Bindungsfähigkeit des VWF an GPIb bestimmt werden. Dazu wird der Testansatz entweder mit Ristocetin und wildtypischem, typischerweise rekombinantem GPIb-Protein oder einem GPIb-Protein-Fragment, das an eine Festphase gebunden ist (WO-A2-01/02853), oder ohne Ristocetin aber mit einem mutierten, typischerweise rekombinantem GPIb-Protein oder einem GPIb-Protein-Fragment, das an eine Festphase gebunden ist, vermischt (WO-A2-2009/007051, WO-A1-2009/026551) und die Bindung des VWF an das GPIb-Protein wird quantifiziert. Alternativ kann die VWF-Aktivität auch anhand der Bindungsfähigkeit des VWF an Collagen bestimmt werden.

In einer anderen bevorzugten Ausführungsform des erfindungsgemäßen Verfahrens wird die Menge oder die Aktivität von Plättchen-assoziiertem Faktor XIII gemessen. Die Messung der Menge von Faktor XIII in einem Testansatz kann mit Hilfe eines heterogenen oder homogenen Bindungstests erfolgen, bei dem mindestens ein Faktor XIII-spezifischer Bindungspartner, bevorzugt ein monoklonaler oder polyklonaler Antikörper oder ein Faktor XIII-bindendes Antikörperfragment verwendet wird. Die Messung der Faktor XIII-Aktivität in einem Testansatz kann mit Hilfe eines Tests bestimmt werden, bei dem der Faktor XIII im Testansatz mit Thrombin in Gegenwart von $Ca^{2+}$-Ionen zu Faktor XIIIa aktiviert wird. Der Testansatz wird dann mit einem synthetischen, glutaminhaltigen Peptid und mit Glycinethylester vermischt, die als Substrate für die Ausbildung intermolekularer Amidbindungen durch Faktor XIIIa dienen. Um den bei dieser Reaktion freigesetzten Ammoniak quantitativ nachzuweisen, wird die Probe zusätzlich mit NADH (Nicotinsäureamid-Adenin-Dinukleotid-Hydrid), NADPH oder Thio-NAD(P)H und mit Komponenten einer NADH-abhängigen Indikatorreaktion vermischt, nämlich mit Glutamatdehydrogenase (GLDH) und α-Ketoglutarat. In Anwesenheit von Ammoniak wandelt GLDH α-Ketoglutarat in Glutamat um. Diese Reaktion verbraucht zusätzlich NADH, und es entsteht NAD+, die oxidierte Form von NADH. NAD+ hat ein anderes Absorptionsspektrum als NADH, so dass sich die Absorption des Testansatzes proportional zum NADH-Verbrauch und damit proportional zur Ammmoniakmenge und damit proportional zur Faktor XIII-Menge bzw. -Aktivität ändert (EP-A2-336353; WO-A1-2011/042071).

In einer anderen bevorzugten Ausführungsform des erfindungsgemäßen Verfahrens wird die Menge von Plättchen-assoziiertem D-Dimer gemessen. Die Messung der Menge von D-Dimer in einem Testansatz kann mit Hilfe eines heterogenen oder homogenen Bindungstests erfolgen, bei dem mindestens ein D-Dimer-spezifischer Bindungspartner, bevorzugt ein monoklonaler oder polyklonaler Antikörper oder ein Faktor D-Dimer-bindendes Antikörperfragment verwendet wird.

## FIGURENBESCHREIBUNG

### Figur 1

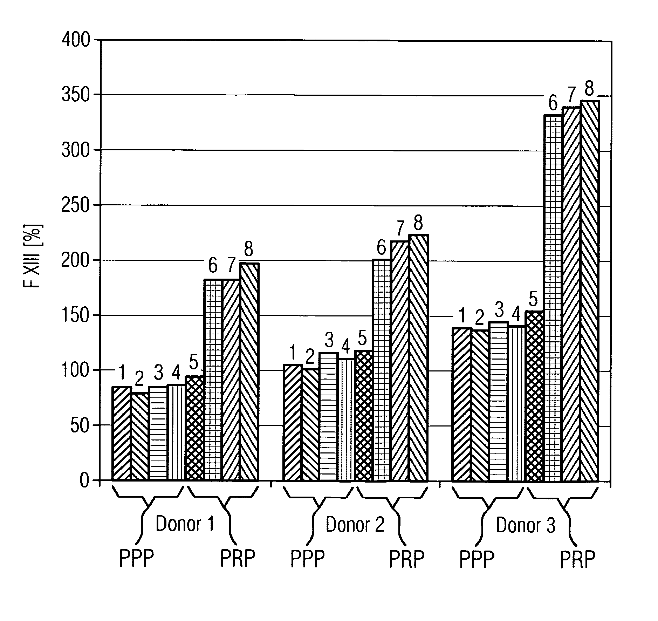

Figur 1 ist eine schematische Darstellung der Faktor XIII-Aktivität (in % der Norm) in Proben von plättchenarmem und plättchenreichem Plasma von 3 gesunden Spendern (Donor 1, 2 und 3) gemäß Ausführungsbeispiel 2. Die Balken 1 - 4 zeigen jeweils die Testergebnisse für eine Probe plättchenarmen Plasmas (PPP-Probe). Die Balken 5 - 8 zeigen jeweils die Testergebnisse für eine Probe plättchenreichen Plasmas (PPR-Probe). Die Balken 1 und 5 repräsentieren Testergebnisse, die in völliger Abwesenheit von Detergenz ermittelt wurden. Die übrigen Balken repräsentieren Testergebnisse, die in Gegenwart von Detergenz (Thesit) im Testansatz ermittelt wurden (2 und 6: Vorbehandlung der Probe mit Thesit/kein Thesit in Testreagenzien; 3 und 7: keine Vorbehandlung der Probe mit Thesit/Thesit in Testreagenzien; 4 und 8: Vorbehandlung der Probe mit Thesit und Thesit in Testreagenzien). Vergleicht man Balken 5 (PRP als Probe und Ermittlung der Testergebnisse in Abwesenheit von Detergenz) mit den Balken 6-8 (PRP als Probe und Ermittlung der Testergebnisse in Gegenwart von Detergenz), so erkennt man, dass eine wesentlich höhere Faktor XIII-Aktivität gemessen wird, die auf die Aktivität von Plättchen-assoziiertem Faktor XIII zurückzuführen ist.

## BEISPIELE

### Beispiel 1: Bestimmung der Aktivität von Plättchen-assoziiertem VWF

Herstellung von plättchenreichem Plasma (PRP):

Jeweils 10 mL frisches Citrat-Vollblut von vier augenscheinlich gesunden Spendern wurden in den Original-Entnahmeröhrchen bei 180 g für 10 Minuten zentrifugiert. Das plättchenreiche Plasma im Überstand wurde mit einer Pipette vorsichtig entnommen und verschlossen bei Raumtemperatur aufbewahrt.

Herstellung von plättchenarmem Plasma (PPP):

Die in den Entnahmeröhrchen verbliebene untere Schicht aus der Herstellung des plättchenreichen Plasmas, die die roten und weißen Blutkörperchen, Reste von Thrombozyten und Plasma enthält, wurde bei 2000 g für 20 Minuten zentrifugiert. Der klare Überstand, d.h. das plättchenarme Plasma, wurde vorsichtig mit einer Pipette entnommen und verschlossen bei Raumtemperatur aufbewahrt.

### Beispiel 1.1: Ristocetin-Kofaktor-Test zur Bestimmung der VWF-Aktivität von Plättchen-assoziiertem VWF

Das oben beschriebene plättchenreiche und plättchenarme Plasma der vier Spender wurde als Probe verwendet. Zur Herstellung eines ersten Testansatzes wurden jeweils 10 $\mu$L Probe mit 20 $\mu$L NaCl-Lösung (0,9 %) und 150 $\mu$L eines Reagenzes enthaltend ca. 1,2 Millionen fixierte Thrombozyten pro $\mu$L, Ristocetin (1,25 mg/mL) und 0,75 Volumenprozent Thesit (Sigma-Aldrich, München, Deutschland) gemischt, so dass sich ein Thesit-Volumenanteil im Testansatz von 0,63 % ergab. Zur Herstellung eines zweiten Testansatzes ohne Detergenz wurden 10 $\mu$L Probe mit 20 $\mu$L NaCl-Lösung (0,9 %) und 150 $\mu$L eines Reagenzes enthaltend ca. 1,2 Millionen fixierte Thrombozyten pro $\mu$L und Ristocetin (1,25 mg/mL) gemischt. Anhand einer Extinktionsmessung wurde die Plättchenaggregation in jedem Testansatz bestimmt. Die Aggregationsreaktion verhält sich proportional zur VWF-Aktivität der Probe.

Die ermittelten VWF-Aktivitäten (VWF:RCo in % der Norm) der 4 plättchenreichen und der 4 plättchenarmen Plasmaproben wurden jeweils gemittelt. Die gemittelten VWF-Aktivitäten ([%], % der Norm) sind in Tabelle 1 dargestellt.

**Tabelle 1: Gemittelte VWF-RCo-Aktivität (% der Norm) von plättchenarmem und plättchenreichem Plasma in Abwesenheit und in Gegenwart eines Detergenzes (Thesit)**

| | VWF:RCo (%) in Abwesenheit von Thesit | VWF:RCo (%) in Gegenwart von Thesit |
|---|---|---|
| **Plättchenarmes Plasma (PPP)** | 89,8 | 95,6 |

(fortgesetzt)

| | VWF:RCo (%) in Abwesenheit von Thesit | VWF:RCo (%) in Gegenwart von Thesit |
|---|---|---|
| **Plättchenreiches Plasma (PRP)** | 95,5 | 121,8 |

Zur Bestimmung der Aktivität von Plättchen-assoziiertem VWF kann nun entweder

a) die Differenz der VWF-Aktivität, die in plättchenreichem Plasma in Gegenwart von Detergenz bestimmt wurde, und der VWF-Aktivität, die in plättchenreichem Plasma in Abwesenheit von Detergenz bestimmt wurde, gebildet werden, also im konkreten Fall:

$$121,8 \text{ \% } - 95,5 \text{ \% } = \underline{26,3 \text{ \%}}$$

oder

b) die Differenz der VWF-Aktivität, die in plättchenreichem Plasma in Gegenwart von Detergenz bestimmt wurde, und der VWF-Aktivität, die in plättchenarmem Plasma in Abwesenheit von Detergenz bestimmt wurde, gebildet werden, also im konkreten Fall:

$$121,8 \text{ \% } - 89,8 \text{ \% } = \underline{32,0 \text{ \%}}$$

oder

c) die Differenz der VWF-Aktivität, die in plättchenreichem Plasma in Gegenwart von Detergenz bestimmt wurde, und der VWF-Aktivität, die in plättchenarmem Plasma in Gegenwart von Detergenz bestimmt wurde, gebildet werden, also im konkreten Fall:

$$121,8 \text{ \% } - 95,6 \text{ \% } = \underline{26,2 \text{ \%}} \text{ .}$$

Die so bestimmte VWF-Aktivität für den Plättchen-assoziierten VWF im Bereich von 26,2 bis 32,0 % (absolut) ist plausibel für gesunde Spender, da für den Plättchen-VWF in der Literatur Werte von 10 - 25 % vom Gesamt-VWF angegeben werden (McGrath, R.T. et al. Platelet von Willebrand factor - structure, function and biological importance. British Journal of Haematology 2010, 148, 834-843) Die um 5,8 % erhöhte VWF-Aktivität, die in plättchenarmem Plasma in Anwesenheit von Thesit gegenüber der Messung in Abwesenheit von Thesit gemessen wird, ist minimal und entweder auf die Impräzision des Tests oder auf einen geringfügigen Einfluss des Detergenzes auf das Testsystem zurückzuführen.

**Beispiel 1.2: Latexagglutinations-Test zur Bestimmung der VWF-Aktivität von Plättchen-assoziiertem VWF**

Bei dem INNOVANCE® VWF Ac Test (Siemens Healthcare Diagnostics Products GmbH, Marburg, Deutschland) handelt es sich um einen Latexagglutinations-Test zur Bestimmung der VWF-Aktivität in Plasmaproben. Das oben beschriebene plättchenreiche und plättchenarme Plasma der vier Spender wurde als Probe verwendet. Zur Bestimmung der VWF-Aktivität wurden jeweils 15 μL Probe, 30 μL Owren's Veronal Puffer, 70 μL Reaktionspuffer (welcher u.a. 0,90 % Volumenanteil des Detergenzes Thesit enthielt ), 13 μL GPIbα-Reagenz (Pufferlösung enthaltend isoliertes GPIbα-Protein, das in Abwesenheit von Ristocetin an VWF bindet) und 40 μL Latexreagenz (Pufferlösung enthaltend Latexpartikel, die mit einem monoklonalen anti-GPIbα-Antikörper beschichtet sind) vermischt, und die Partikelagglutination wurde turbidimetrisch bestimmt. In den Testansätzen befand sich somit ein Volumenanteil von 0,38 % Thesit. Die ermittelten VWF-Aktivitäten (in % der Norm) der 4 plättchenreichen und der 4 plättchenarmen Plasmaproben wurden jeweils gemittelt. Die gemittelten VWF-Aktivitäten ([%], % der Norm) sind in Tabelle 2 dargestellt.

**Tabelle 2: Gemittelte VWF-Aktivität (% der Norm) von plättchenarmem und plättchenreichem Plasma in Gegenwart eines Detergenzes (Thesit)**

|  | VWF (%) in Anwesenheit von Thesit |
|---|---|
| **Plättchenarmes Plasma (PPP)** | 102, 9 |
| **Plättchenreiches Plasma (PRP)** | 121,8 |
| **Plättchen-assoziierter VWF** | 18, 9 |

Die Differenz der VWF-Aktivität, die in plättchenreichem Plasma in Gegenwart von Detergenz bestimmt wurde, und der VWF-Aktivität, die in plättchenarmem Plasma in Gegenwart von Detergenz bestimmt wurde, also im konkreten Fall:

$$121{,}8~\% - 102{,}9~\% = \underline{18{,}9~\%}$$

entspricht dem Plättchen-assoziierten VWF.

**Beispiel 2: Bestimmung der Aktivität von Plättchen-assoziiertem Faktor XIII**

Zur Bestimmung der F XIII-Aktivität wurde der Berichrom® FXIII Test(Siemens Healthcare Diagnostics Products GmbH, Marburg, Deutschland) verwendet. In der Probe enthaltener F XIII wird durch Zugabe von Thrombin zur Probe zu F XIIIa aktiviert. Ebenfalls durch Thrombin gebildetes Fibrin beschleunigt diese Reaktion. Fibrinogen wird vor der Messung nicht entfernt, da dies mit einem Verlust an F XIII verbunden ist. Stattdessen wird durch Thrombineinwirkung gebildetes Fibrin durch ein aggregationshemmendes Peptid an der Ausbildung eines Gerinnsels gehindert und in Lösung gehalten. F XIIIa verknüpft ein spezifisches Peptidsubstrat mit Glycinethylester unter Freisetzung von Ammoniak. Dieser wird in einer parallel ablaufenden enzymatischen Reaktion bestimmt. Gemessen wird die Abnahme an NADH über die Extinktion bei 340 nm. Wenn Ergebnisse oberhalb des initialen Messbereichs lagen, wurde eine 1:2 Vorverdünnung der Probe in einem Phospatpuffer durchgeführt und erneut gemessen.

Das oben beschriebene plättchenreiche und plättchenarme Plasma von drei gesunden Spendern wurde als Probe verwendet. Es wurden jeweils 15 µL Probe mit 75 µL Berichrom FXIII Aktivatorreagenz (enthaltend unter anderem Thrombin und mit 1,49 Volumenprozent Thesit oder ohne Thesit) gemischt und inkubiert. Danach wurden 75 µL des Berichrom FXIII Nachweisreagenzes (enthaltend unter anderem GLDH (20 IU/mL), ein synthetisches F XIII-Peptidsubstrat (2,4 g/L), ADP, Glycinethylester (1,4 g/L) und $\alpha$-Ketoglutarat (2.7 g/L)) die Farbreaktion gestartet. Somit ergab sich ein Volumenanteil von 0,68 % Thesit im Testansatz.

In einem zusätzlichen Experiment wurden die Proben mit Detergenz vorbehandelt. Dazu wurden die Proben in Gegenwart eines Volumenanteils von 0,63 % Thesit für 30 Minuten bei 37 °C inkubiert, bevor sie mit den Reagenzien zur Bestimmung der Faktor XIII-Aktivität vermischt wurden.

Die ermittelten Faktor XIII-Aktivitäten (in % der Norm) der plättchenreichen und der plättchenarmen Plasmaproben der 3 Spender sind in Figur 1 dargestellt.

Die Faktor XIII-Aktivität der PPP-Probe eines Spenders - unabhängig davon mit welchem Testansatz die Aktivität bestimmt wurde- und die Faktor XIII-Aktivität der PRP-Probe eines Spenders in Abwesenheit von Detergenz ist jeweils sehr ähnlich. Dies belegt, dass weder die Gegenwart von Detergenz, noch die Vorbehandlung mit Detergenz und ebensowenig die Gegenwart von Thrombozyten im Testansatz den Faktor XIII-Test stören. Sowohl die Vorbehandlung einer PRP-Probe mit Detergenz als auch die Verwendung von Detergenz in einem Testreagenz -kurz: immer wenn das Testverfahren in Gegenwart von Detergenz durchgeführt wird- wird in plättchenreichem Plasma eine große Menge von Plättchen-assoziiertem Faktor XIII freigesetzt. Dies zeigt, dass das erfindungsgemäße Verfahren die gesamte Menge an freisetzbarem Plättchen-assoziiertem Faktor XIII zusätzlich zum Plasma-Faktor XIII erfasst.

Der Plättchen-assoziierte Anteil an Faktor XIII kann durch Bildung der Differenz der Gesamtmenge und des Plasma-Anteils ermittelt werden. Dadurch ergeben sich für die 3 gemessenen Proben die Werte gemäß Tabelle 3 für den Plättchen-assoziierten Faktor XIII-Anteil.

**Tabelle 3: Faktor XIII-Aktivitäten (% der Norm) von Plättchen-assoziiertem Faktor XIII durch Differenzbildung der Messwerte eines ersten und diverser alternativer zweiter Testansätze**

| | Gesamt-Faktor XIII (%) | | |
| --- | --- | --- | --- |
| | Spender 1 | Spender 2 | Spender 3 |
| PRP als Probe, Test mit Detergenz | 181,6 | 217,4 | 312,6 |

| | Plättchen-assoziierter Faktor XIII (%) | | |
| --- | --- | --- | --- |
| | Spender | Spender 1 2 3 | Spender |
| Plasma (PPP) als Probe, Test ohne Detergenz | 97.8 | 112.7 | 173.7 |
| Plasma (PPP) als Probe, Test mit Detergenz | 98.2 | 101.2 | 169.3 |
| PRP als Probe (PPP), Test ohne Detergenz | 87.2 | 99.7 | 161.0 |

Der Gehalt an Plättchen-assoziiertem Faktor XIII ist beim Spender Nr. 3 erheblich höher als bei den Spendern Nr. 1 und 2.

**Patentansprüche**

1.  Verfahren zur Bestimmung der Menge oder der Aktivität eines Plättchen-assoziierten Analyten in einer Probe eines Individuums, **dadurch gekennzeichnet, dass** das Verfahren folgende Schritte umfasst:

    a) Messung der Menge oder der Aktivität des Analyten in einem ersten Testansatz enthaltend plättchenreiches Plasma des Individuums und ein Detergenz und in einem zweiten Testansatz enthaltend entweder

    i) plättchenreiches Plasma des Individuums und kein Detergenz oder
    ii) plättchenarmes Plasma des Individuums und kein Detergenz oder
    iii) plättchenarmes Plasma des Individuums und ein Detergenz, und

    b) Vergleich der Messergebnisse,

    wobei die Differenz zwischen den Messergebnissen der Menge oder der Aktivität des Plättchen-assoziierten Analyten entspricht.

2.  Verfahren nach Anspruch 1, wobei das Detergenz ein nichtionisches Tensid ist, bevorzugt aus der Gruppe Polyethylenglycol-[4- (1,1,3,3-tetramethylbutyl) phenyl]-ether (TRITON X-100), Polyoxyethylen(20)-sorbitanmonolaurat (TWEEN 20) und Polidocanol (THESIT).

3.  Verfahren nach einem der vorherigen Ansprüche, wobei das Detergenz im Testansatz in einer Endkonzentration mit einem Volumenanteil von 0,1 bis 1,6 %, bevorzugt in einer Endkonzentration in einem Volumenanteil von 0,2 bis 0,8 % im Testansatz enthalten ist.

4.  Verfahren nach einem der vorherigen Ansprüche, wobei die Messung der Menge oder der Aktivität des Analyten im ersten und im zweiten Testansatz erfolgt, indem jeder Testansatz mit einer partikulären Festphase, bevorzugt mit Latexpartikeln, vermischt wird, die mit mindestens einem Analyt-spezifischen Bindungspartner assoziiert ist und wobei die Agglutination der partikulären Festphase gemessen wird.

5.  Verfahren nach einem der vorherigen Ansprüche zur Bestimmung der Menge oder der Aktivität eines Plättchen-assoziierten Analyten aus der Gruppe von Willebrand Faktor, Faktor XIII, D-Dimer, Fibrinogen und Polyphosphate.

**Claims**

1. Method for determining the amount or the activity of a platelet-associated analyte in a sample from an individual, **characterized in that** the method comprises the following steps:

   a) measuring the amount or the activity of the analyte in a first assay volume containing platelet-rich plasma from the individual and a detergent and in a second assay volume containing

      i) platelet-rich plasma from the individual and no detergent or
      ii) platelet-poor plasma from the individual and no detergent or
      iii) platelet-poor plasma from the individual and a detergent, and

   b) comparing the measurement results,

   wherein the difference between the measurement results corresponds to the amount or the activity of the platelet-associated analyte.

2. Method according to Claim 1, wherein the detergent is a nonionic surfactant, preferably from the group consisting of 4-(1,1,3,3-tetramethylbutyl)phenyl-polyethylene glycol (TRITON X-100), polyoxyethylene (20) sorbitan monolaurate (TWEEN 20) and polidocanol (THESIT).

3. Method according to either of the preceding claims, wherein the detergent is present in the assay volume in a final concentration at a proportion by volume of from 0.1 to 1.6%, preferably in a final concentration in a proportion by volume of from 0.2 to 0.8%.

4. Method according to any of the preceding claims, wherein the amount or the activity of the analyte in the first and in the second assay volume is measured by mixing each assay volume with a particulate solid phase, preferably with latex particles, which is associated with at least one analyte-specific binding partner, and wherein the agglutination of the particulate solid phase is measured.

5. Method according to any of the preceding claims for determining the amount or the activity of a platelet-associated analyte from the group consisting of von Willebrand factor, factor XIII, D-dimer, fibrinogen and polyphosphates.

**Revendications**

1. Procédé de détermination de la quantité ou de l'activité d'un analyte associé aux plaquettes d'un échantillon d'un individu, **caractérisé en ce que** le procédé comprend les stades suivants :

   a) mesure de la quantité ou de l'activité de l'analyte dans une première préparation de test contenant du plasma riche en plaquettes de l'individu et un détergent et dans une deuxième préparation de test contenant soit

      i) du plasma riche en plaquettes de l'individu et pas de détergent ou
      ii) du plasma pauvre en plaquettes de l'individu et pas de détergent ou
      iii)du plasma pauvre en plaquettes de l'individu et un détergent et

   b) comparaison des résultats des mesures

   dans lequel la différence entre les résultats des mesures correspond à la quantité ou à l'activité de l'analyte associé aux plaquettes.

2. Procédé suivant la revendication 1,
   dans lequel le détergent est un agent tensioactif non ionique, de préférence choisi dans le groupe du polyéthylèneglycol -[4-(1, 1, 3, 3-tétraméthylbutyl) phényl]- éther (TRITON X-100), du monolaurate de polyoxyéthylène (20) sorbitan (TWEEN 20) et du polidocanol (THESIT).

3. Procédé suivant l'une des revendications précédentes, dans lequel le détergent dans la préparation de test est contenu en une concentration finale ayant une proportion en volume de 0,1 à 1,6%, de préférence en une concentration finale ayant une proportion en volume de 0,2 à 0,8% dans la préparation de test.

4. Procédé suivant l'une des revendications précédentes, dans lequel on effectue la mesure de la quantité ou de l'activité de l'analyte dans la première et dans la deuxième préparations de test, en mélangeant chaque préparation de test à une phase solide particulaire, de préférence à des particules de latex, qui est associée à au moins un partenaire de fixation spécifique à l'analyte et dans lequel on mesure l'agglutination de la phase solide particulaire.

5. Procédé suivant l'une des revendications précédentes de détermination de la quantité ou de l'activité d'un analyte associé à des plaquettes choisies dans le groupe du facteur de Willebrand, du facteur XIII, du D-Dimer, du fibrinogène et du polyphosphate.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 515194 A2
- US 20120164644 A1
- WO 0102853 A2
- WO 2009007051 A2
- WO 2009026551 A1
- EP 336353 A2
- WO 2011042071 A1

**In der Beschreibung aufgeführte Nicht-Patentliteratur**

- **DE ROMEUF, C. ; MAZURIER, C.** Interest of a simple and fast method for platelet von Willebrand factor characterization. *Thrombosis Research,* 1996, vol. 83 (4), 287-298
- **RODEGHIERO, F. et al.** Platelet von Willebrand factor assay: results using two methods for platelet lysis. *Thrombosis Research,* 1990, vol. 59, 259-267
- **THUY, L.P. et al.** Identification of platelet receptors for bovine von Willebrand factor on human platelets by a new platelet receptor test. *Biochimica et Biophysica Acta,* 1981, vol. 678, 187-193
- **MCGRATH, R.T. et al.** Platelet von Willebrand factor - structure, function and biological importance. *British Journal of Haematology,* 2010, vol. 148, 834-843